# EUROPÄISCHE PATENTSCHRIFT

(11) **EP 0 825 211 B1**
(45) Veröffentlichungstag und Bekanntmachung des Hinweises auf die Patenterteilung: **25.02.2004**
(21) Anmeldenummer: 97113525.6
(22) Anmeldetag: 06.08.1997
(51) Int. Cl.: C08G 18/78

(54) **Verfahren zur Herstellung von aliphatischen und/oder cycloaliphatischen und/oder araliphatischen Isocyanatgruppen aufweisenden Allophanaten**
Process for the preparation of allophanates containing aliphatic and/or cycloaliphatic and/or araliphatic isocyanate groups
Procédé pour la préparation d'allophanates contenant des groupes isocyanate aliphatiques et/ou cycloaliphatiques et/ou araliphatiques

(30) Priorität: 19.08.1996 DE 19632951
(43) Veröffentlichungstag der Anmeldung: 25.02.1998
(73) Patentinhaber: BAYER AG, 51368 Leverkusen (DE)
(72) Erfinder: König, Klaus, Dr., 51519 Odenthal (DE); Reiff, Helmut, Dr., 51375 Leverkusen (DE); Wamprecht, Christian, Dr., 41472 Neuss (DE); Träubel, Harro, Dr., 51373 Leverkusen (DE)

(56) Entgegenhaltungen:
- EP-A- 0 142 838
- EP-A- 0 144 841
- FR-A- 2 119 998
- GB-A- 1 145 952

## Beschreibung

Die Erfindung betrifft ein neues Verfahren zur Herstellung von aliphatischen und/oder cycloaliphatischen und/oder araliphatischen Isocyanatgruppen aufweisenden Allophanaten.

Isocyanatgruppen aufweisende Allophanate sind bekannt. Sie werden z.B. nach GB-A 994 890 durch Umsetzung von Urethangruppen aufweisenden Isocyanaten mit überschüssigen Mengen an Diisocyanaten erhalten, entweder thermisch oder in Gegenwart von in der Polyurethanchemie üblichen Katalysatoren wie Metallchelaten, Metallcarboxylaten oder tertiären Aminen.

Nachteilig sind insbesondere bei den (cyclo)aliphatischen Polyisocyanaten sehr lange Reaktionszeiten bei 130-135°C (Vergilbung) oder im Falle der Katalyse beträchtliche Verunreinigungen an Isocyanatgruppen aufweisenden Uretdionen, Isocyanuraten und nicht umgesetzten Urethanen.

Bekannt ist weiterhin ein Verfahren zur Herstellung von (cyclo)aliphatischen Isocyanatgruppen aufweisenden Allophanaten (vgl. DE-A 2 729 990) durch Umsetzung von überschüssigen Mengen von (cyclo)aliphatischen Polyisocyanaten mit Urethangruppen aufweisenden (cyclo)aliphatischen Isocyanaten in Gegenwart von starken Säuren, die mit (cyclo)aliphatischen Isocyanaten ein gemischtes Carbamidsäureanhydrid bilden.

Nachteilig sind in diesem Fall ein verbleibender Gehalt an Restmonomer und der saure Katalysator, der beispielsweise mit Propylenoxid entfernt werden muß, was einen aufwendigen zusätzlichen Reaktionsschritt bedingt.

Aus der DE-A 2 165 023 ist bekannt, daß man bei der Umsetzung von Isocyanatgruppen aufweisenden Oxadiazintrionen der Formel (II) (s. Seite 3) mit aktiven Wasserstoffverbindungen wie Alkoholen in einem ersten Reaktionsschritt die beiden endständigen Isocyanatgruppen zu einem Urethangruppen aufweisenden Oxadiazintrion umsetzt und dann in einem zweiten, sehr langsamen Schritt den Oxadiazintrionring zum Allophanat öffnet, wobei dieser zweite Reaktionsschritt durch ganz spezielle Katalysatoren vom Typ Tetraalkyl-distannoxan beschleunigt wird.

Es hat nicht an Versuchen gefehlt, die Nachteile der bekannten Verfahren zu überwinden. Diese Aufgabe konnte nun durch ein Verfahren gelöst werden.

Es wurde nun gefunden, daß man den zweiten Reaktionschritt zuerst machen und den ersten Reaktionsschritt fast völlig unterbinden kann, wenn man erfindungsgemäß Isocyanatgruppen aufweisende Oxadiazintrione in Gegenwart von basischen Katalysatoren mit primären oder sekundären monofunktionellen Alkoholen umsetzt.

Gegenstand der Erfindung ist ein Verfahren zur Herstellung von (cyclo)aliphatischen und/oder araliphatischen Isocyanatgruppen aufweisenden (Cyclo)Allophanaten der Formel (I) 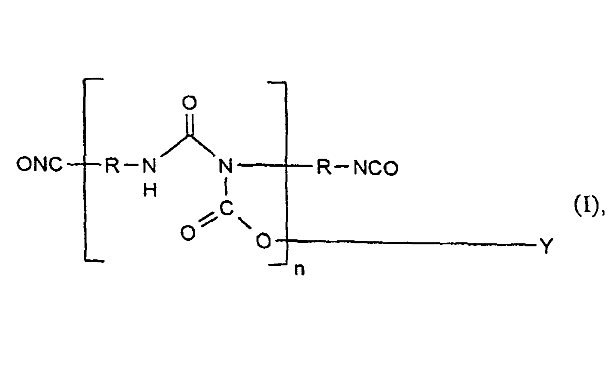 in welcher
- R: denjenigen Rest bedeutet, wie er durch Abstraktion der Isocyanatgruppen von (cyclo)aliphatischen und araliphatischen Diisocyanaten entsteht,
- Y: für den ein Rest eines monofunktionellen Alkohols steht und
- n: für die Zahl 1, 2, 3, 4 und 5 steht,
dadurch gekennzeichnet, daß man Isocyanatgruppen aufweisende (Oligo)Oxadiazintrione der Formel (II) 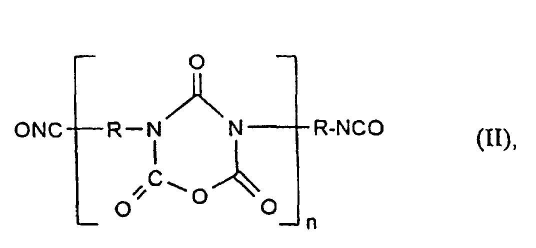 in welcher
- R und n: die bei Formel (I) angegebene Bedeutung haben
in Gegenwart von basischen Katalysatoren mit primären oder sekundären monofunktionellen Alkoholen umsetzt.

Die Oxadiazintrione der Formel (II) enthalten gegebenenfalls Ether-, Ester-, Carbonat-, Thioether- oder Amid-Gruppen. Erfindungsgemäß werden sie mit primären- und/oder sekundären monofunktionellen Alkoholen Y(OH) des Molgewichtsbereiches von 32 - 6000 bei einer Temperatur von 0° - 60° C in Gegenwart von basischen Katalysatoren eines pKa- Wertes von > 6, vorzugsweise > 7,5 umgesetzt.

In Formeln (I) und (II) steht der Rest R vorzugsweise für einen gegebenfalls durch Ethergruppen unterbrochenen und/oder mit Methylgruppen substituierten geradkettigen aliphatischen Rest mit 4-12 Kohlenstoffatomen.

Die erfindungsgemäß eingesetzten Alkohole sind vorzugsweise primäre aliphatische monofunktionellen Alkohole des Molgewichtsbereiches 32 - 6000.

Als Katalysatoren werden tertiäre, gegebenfalls cyclische, gegebenfalls heterocyclische Amine eines pKa-Wertes von >6, vorzugsweise >7,5 und die Alkali(Na, K) und Erdalkali(Mg, Ca)-Salze von Kohlensäure, Mono-, Di- und Tricarbonsäuren, Alkoholate oder Phenolate verwendet.

Isocyanatgruppen aufweisende Oxadiazintrione sind bekannt und werden z B. gemäß der GB-A 1 145 952 aus aliphatischen Polyisocyanaten und Kohlendioxid in Gegenwart von organischen Verbindungen des dreiwertigen Phosphors wie beispielsweise Tributylphosphin oder Trioctylphosphin hergestellt. In der Regel wird das überschüssig verwendete Diisocyanat durch Dünnschichtdestillation völlig entfernt.

Es ist überraschend, daß die Reaktion im Allgemeinen bereits bei 10 - 30°C fast vollständig abläuft, was man an der lebhaften Kohlendioxidabspaltung leicht feststellen kann. Im allgemeinen ist die Reaktion bereits nach 0,5 - 2,5 Stunden abgeschlossen. Gemäß Stand der Technik sind zur Herstellung von (cyclo)aliphatischen Diisocyanatoallophanaten dagegen Temperaturen von 110 - 140°C erforderlich.

Ein weiterer Vorteil des erfindungsgemäßen Verfahrens ist, daß die (cyclo)aliphatischen Diisocyanato-allophanate sofort monomerenfrei anfallen. Der sonst übliche Vorgang des Dünnschichtverdampfens entfällt. Zwar liegen die als Ausgangssubstanzen eingesetzten Diisocyanato-oxatriazinone in der Regel als gedünnschichtete Harze vor, jedoch erhält man nach dem erfindungsgemäßen Verfahren ein wesentlich günstigeres Produkt-/Destillat-Verhältnis und vermeidet in jedem Falle das Entstehen von mit Urethangruppen kontaminierten Destillaten (monomeren Diisocyanaten).

Ein wesentlicher Vorteil des erfindungsgemäßen Verfahrens besteht darin, daß man aus ein und demselben Diisocyanato-oxadiazintrion eine beliebige Anzahl von Polyisocyanato-allophanaten herstellen kann, die je nach Molekulargewicht der Hydroxylverbindung viele Variationen ermöglichen.

Ausgangsmaterialien für das erfindungsgemäße Verfahren zur Herstellung von aliphatischen und/oder cycloaliphatischen Isocyanatgruppen aufweisenden Allophanaten sind
- A.: Hydroxyl verbindungen
- B.: Diisocyanato-oxadiazintrione der allgemeinen Formel (II) sowie
- C: Basische Katalysatoren

### A: Hydroxylverbindungen

Als Hydroxylverbindungen Y (OH) steht, können sowohl Phenole wie z.B. Phenol, α-Naphthol, Kresol, Resorcin oder Trishydroxybenzole als auch alkoholische Hydroxylgruppen aufweisende aliphatische Verbindungen eingesetzt werden. Derartige alkoholische Gruppen aufweisende Verbindungen sind gegenüber den beispielhaft genannten Phenolen bevorzugt.

Zu diesen bevorzugten alkoholischen Hydroxylverbindungen Y (OH)ₘ gehören
1. Niedermolekulare, gegebenenfalls Etherbrücken aufweisende monofunktionelle aliphatische Alkohole des Molekulargewichtsbereichs 32 - 250 wie z.B. Methanol, Ethanol, Propanol, Isopropanol, isomere Butanole, Allylalkohol, Pentanole, Hexanole und Heptanole, 2-Ethylhexanol, Fettalkohole mit 10-20 Kohlenstoffatomen;
2. Cycloaliphatische monofunktionelle Alkohole des Molekulargewichtsbereichs 88-250, wie z.B. Cyclopentanol, Cyclohexanol, Methylcyclohexanol, Trimethylcyclohexanol, 4-tert.-Butylcyclohexanol, Menthol, Borneol und Isoborneol, 2-Hydroxydecalin;
3. Aratiphatische monofunktionelle Alkohole des Molekulargewichtsbereichs 103-300, wie z.B. Benzylalkohol, Phenylethylalkohol, 3-Phenylpropanol oder
4. Eine Hydroxylgruppe aufweisende Polythioether, Polyacetale, Polycarbonate oder insbesondere Polyester bzw. Polyether der in der Polyurethanchemie an sich bekannten Art mit mittleren Molekulargewichten von 250 bis 8000, vorzugsweise 300 bis 3000. Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren.
   Anstelle der freien Polycarbonsäuren können auch die entsprechenden Polycarbonsäureanhydride oder entsprechenden Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein. Als Beispiele hierfür seien genannt:
   Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthatsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester, Terephthalsäure-bis-glykolester. Als mehrwertige Alkohole kommen z.B. Ethylenglykol, Propylenglykol-(1,2) und -(1,3), Butylenglykol-(1,4) und-(2,3), Hexandiol-(1,6), Octandiol-(1,8), Neopentylglykol, Cyclohexandimethanol(1,4-bis-hydroxymethylcyclohexan), 2-Methyl-1,3-propandiol, Glycerin, Trimethylolpropan, Hexantriol-(1,2,6), Butantriol-(1,2,4), Trimethylolethan, Pentaerythrit, Chinit, Mannit und Sorbit, Methylglykosid, ferner Diethylenglykol, Triethylenglykol, Tetraethylenglykol, Polyethylenglykole, Dipropylenglykol, Polypropylenglykole, Dibutylenglykole und Polybutylenglykole in Frage.
   Auch Polyester aus Lactonen, z.B. ε-Caprolacton oder Hydroxycarbonsäuren, z.B. ω-Hydroxycapronsäure, sind einsetzbar.

Auch die erfindungsgemäß in Frage kommenden, eine Hydroxylgruppe aufweisenden Polyether, sind an sich bekannt und werden z.B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von BF₃, oder durch Anlagerung dieser Epoxide, gegebenenfalls im Gemisch oder nacheinander, an Startkomponenten mit reaktionsfähigen Wasserstoffatomen wie Alkohole oder Phenole, z.B. Wasser, Ethylenglykol, Propylenglykol-(1,3) oder -(1,2), Trimethylolpropan, 4,4'-Dihydroxydiphenylpropan, hergestellt.

Unter den Polythioethern seien insbesondere die Kondensationsprodukte von Thiodiglykol mit sich selbst und/oder mit anderen Glykolen, Dicarbonsäuren oder Formaldehyd angeführt. Je nach den Co-Komponenten handelt es sich bei den Produkten um Polythiomischether, Polythioetherester oder Polythioether-polyacetate.

Als Polyacetale kommen z.B. die aus Glykolen, wie Diethylenglykol, Triethylenglykol, 4,4'-Dioxethoxy-diphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diethylenglykol, Triethylenglykol, Tetraethylenglykol mit Diarylcarbonaten, z.B. Diphenylcarbonat oder Phosgen, hergestellt werden können.

Die unter 1. genannten einfachen primären aliphatischen Alkohole sowie die unter 4. genannten Polyester- bzw. Polyetherpolyole werden beim erfindungsgemäßen Verfahren bevorzugt eingesetzt.

Besonders bevorzugt beim erfindungsgemäßen Verfahren werden langkettige, primare C₈-C₃₆-Fettalkohole sowie Polyethylenglykol-mono-C₁-C₆-alkylether wie beispielsweise die Carbowachse (Polyethylenglykol-mononmethylether vom Molgewicht 350, 500, 750 und 1000) sowie tertiären Stickstoff enthaltende Mono Alkohole wie beispielsweise Dimethylaminoethanol, N-Methyldiethanolamin, Triethanolamin und Morpholinoethanol.

Selbstverständlich können auch Mischungen der vorstehend genannten Hydroxylverbindungen eingesetzt werden. Dies ist sogar eine bevorzugte Ausführungsform des erfindungsgemäßen Verfahrens.

### B: Oxadiazintrione

Als Oxadiazintrione können alle bekannten Produkte verwendet werden, sofern diese eine oder mehrere NCO-Gruppen im Molekül enthalten. Insbesondere die Diisocyanate sind bevorzugte Ausgangsmaterialien für das erfindungsgemäße Verfahren.

Beispielhaft genannt für die bevorzugten Produkte mit der allgemeinen Formel seien diejenigen, in denen R der zweiwertige Rest von aliphatischen - oder cycloaliphatischen - oder araliphatischen - Diisocyanaten bedeutet, nachdem die beiden NCO-Gruppen abstrahiert worden sind, d.h. Produkte auf der Basis Ethylendiisocyanat, 1,4-Diisocyanatobutan, 1,6-Diisocyanato-hexan, Trimethylhexandiisocyanat, 1,3- und 1,4-Bis-isocyanatomethyl-cyclohexan, Isophorondiisocyanat, 4,4'-Diisocyanato-dicyclohexylmethan, sowie die Ethergruppen-haltigen ωω'-Diisocyanate wie sie beispielsweise von Diethylether, Dipropylether oder Dibutylether abgeleitet sind und die araliphatischen Diisocyanate 1,3- und 1,4-Xylylendiisocyanate (XDI der Fa. Takeda, Japan).

Bei all diesen Oxadiazintrionen können als Nebenprodukte solche der allgemeinen Formel (II) 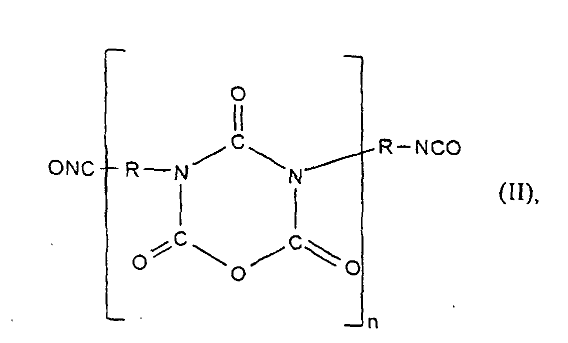 vorhanden sein, in der R die oben angegebene Bedeutung hat und n eine ganze Zahl, deren Wert 1, 2, 3, 4 oder 5 ist bedeutet. Bevorzugte Mischungen sind solche, in denen n im Mittel 1-1,3 beträgt.

### C: Katalysatoren

Als Katalysatoren können fast alle basischen Verbindungen eingesetzt werden; bevorzugt werden Verbindungen mit einem pKa-Wert von größer 6, besonders bevorzugt werden Verbindungen mit einem pKa-Wert von größer 7,5 verwendet.

Bevorzugte Katalysatoren sind tertiäre Amine wie Trimethylamin, Triethylamin, Tributylamin, Dimethylbenzylamin, 1,4-Diazabicyclooctan, 1,5-Diazabicyclo-nonen sowie Tetramethylbutandiamin, Tetramethylpropandiamin und bis-N-Dimethylaminoethylether.

Weitere für das erfindungsgemäße Verfahren geeignete und bevorzugte Katalysatoren sind die Alkali- und Erdalkalisalze von Carbonsäuren wie Kohlensäure, Ameisensäure, Essigsäure, Propionsäure sowie der ggf. substituierten Benzoesäuren. Auch Phenolate wie Natriumphenolat oder Alkoholate wie Natriummethylat sind geeignete Katalysatoren.

Auch Stickstoff-haltige, aromatische Verbindungen wie Pyridin, Mono-C₁-C₄alkylpyridine, Dimethylpyridine, N- Dimethylamino- pyridine, Diethylpyridine und Trimethylpyridin sind Katalysatoren , sofern ihr pKa-Wert größer 6 ist. Für das erfindungsgemäße Verfahren brauchbare Katalysatoren sind ferner C₁-C₄-N-Alkylpyrrole, -pyrroline, -pyrrolidine, pyrazole, -imidazole, -imidazoline, -imidazolidine, -1,2,3-triazole, -1,2,4-triazole, weiterhin die ggf. alkylierten Pyrimidine, Pyridazine, 1,2,3-, 1,2,4-, 1,3,5-Triazine sowie die ggf. alkylierten Chinoline, Isochinoline, Chinoxaline und Acridine.

Das erfindungsgemäße Verfahren wird in der Regel lösungsmittelfrei durchgeführt. In speziellen Fallen - beispielsweise bei Verwendung von unlöslichen Katalysatoren - kann es vorteilhaft sein, organische Lösungsmittel wie beispielsweise Benzol, Toluol, Xylol, Aceton, Methylethylketon, Methylisobutylketon, Tetrahydrofuran, N-Methylpyrrolidon, Dimethylacetamid, Sulfon, Dimethylformamid, Ethylacetat oder Butylacetat mitzuverwenden. Die Menge wird in der Regel so bemessen, daß 30 - 95%ige Lösungen der Endprodukte entstehen

Die Durchführung des erfindungsgemäßen Verfahrens ist denkbar einfach. Im allgemeinen wird das Isocyanatgruppen aufweisende Oxadiazintrion vorgelegt und bei -20 bis 60°C, vorzugsweise bei Temperaturen von 10 bis 40° C eine Mischung aus dem Katalysator und der ungefähr equivalenten Menge der alkoholischen Komponente zugetropft. Die Katalysatormenge beträgt im allgemeinen 0,25 bis 2,5%. Die Reihenfolge kann selbstverständlich umgekehrt werden; man kann aber auch das Isocyanatgruppen-haltige Oxadiazintrion mit dem Katalysator mischen und dann die Hydroxylkomponente zutropfen. Nach 0,5 bis 5,0 Stunden ist die Reaktion im allgemeinen beendet und man rührt noch kurze Zeit bei 40 - 60°C nach.

Das Ende der Reaktion ist erreicht, wenn die Gasabspaltung beendet ist. Man kann den Endpunkt der Reaktion auch z.B. titrimetrisch (NCO-Bestimmung) oder aber IR-spektroskopisch (Verschwinden der Ring-Bande) bestimmen.

Bei Reaktionsende liegen die gewünschten erfindungsgemäß hergestellten Allophanate vor. Wenn erforderlich, werden die Katalysatoren entfernt. Heterogene Katalysatoren können einfach abfiltriert werden, leicht flüchtige Amine kann man abdestillieren (im Vakuum) und schwerflüchtige Katalysatoren können beispielsweise durch equivalente Mengen an Säuren wie zum Beispiel Phosphorsäure, Salzsäure, p-Toluolsulfonsäure oder Methansulfonsäure neutralisiert oder desaktiviert werden.

Bei der Durchführung des erfindungsgemäßen Verfahrens werden im allgemeinen Art und Mengenverhältnisse der Ausgangsmaterialien so gewählt, daß mindestens zwei Isocyanatgruppen aufweisende Allophanate, d.h. Allophanatpolyisocyanate als Verfahrensprodukte entstehen. Derartige erfindungsgemäße Verfahrensprodukte zeichnen sich durch eine hervorragende thermische Stabilität aus.

Das erfindungsgemäße Verfahren eignet sich auch für eine kontinuierliche Durchführung. Hierbei werden zweckmäßigerweise mehrere Reaktoren in Form einer Kaskade hintereinandergeschaltet. In den ersten Reaktor werden kontinuierlich Diisocyanato-oxatriazinon, Hydroxylverbindung und Katalysator eindosiert. Durch Einstellung von Temperatur und Durchsatz wird erreicht, daß die Reaktion bei Verlassen des letzten Reaktors vollständig ist

Die erfindungsgemäß herstellbaren Allophanatpolyisocyanate können zur Herstellung von Polyurethan-Schaumstoffen, Elastomeren, Duromeren, Beschichtungen und Verklebungen eingesetzt werden.

Besonders geeignet sind sie als Rohstoffe für hochwertige, lichtstabile, wetterfeste Lackierungen eventuell in Kombination mit hydroxylfunktionellen höhermolekularen Verbindungen. Hierbei zeichnen sich die Allophanatpolyisocyanate im Vergleich zu Polyisocyanaten mit Urethan-, Biuret- oder Isocyanuratstruktur durch ihre gute Verträglichkeit mit handelsüblichen Polyacrylaten aus.

Ein weiterer Vorteil des erfindungsgemäßen Verfahrens ist in den vielfältigen Variationsmöglichkeiten insbesondere bezüglich Art und Mengenverhältnis der preiswerten Ausgangsmaterialien (Hydroxylgruppen aufweisende Verbindungen) zu sehen. So kann z.B. die NCO-Funktionalität der erfindungsgemäßen Verfahrensprodukte je nach Wahl der Hydroxylverbindung in weiten Grenzen gesteuert werden. Der Einsatz von Fettalkoholen ergibt Produkte mit guter Benzinlöslichkeit. Durch Verwendung von cycloaliphatischen oder aromatischen Hydroxylverbindungen wird eine hervorragende Härte der Lackierungen erreicht.

Insbesondere ist auch die ausgezeichnete Lagerstabilität der erfindungsgemäß hergestellten Allophanatpolyisocyanate hervorzuheben. Die erfindungsgemäßen Verfahrensprodukte zeigen keinerlei Tendenz zur Abspaltung von monomerem Ausgangsisocyanat und unterscheiden sich insbesondere in diesem Punkt vorteilhaft von den bekannten Biuretgruppen aufweisenden Polyisocyanaten.

### Beispiele

Polyisocyanat 1: Auf Hexamethylendiisocyanat-Basis hergestelltes Diisocyanato-oxadiazintrion vom NCO-Gehalt 21,0 Gew.-% und einer Viskosität von 2500 mPas (Baymicron OxaWM 06, Bayer AG).
Polyisocyanat 2: Über einen Kurzwegverdampfer gereinigtes Polyisocyanat 1 vom NCO-Gehalt 22,5 Gew.-%, ein farbloses Harz der Viskosität 2100 mPas.
Polyisocyanat 3: Diisocyanato-oxadiazintrion auf Basis Xylylendiisocyanat, hergestellt aus Xylylendiisocyanat, Kohlendioxid und dem Katalysator Tributylphosphin, ein hochvishoses , gelbbraunes Harz vom NCO-Gehalt 17,3 Gew.-%.

### Beispiel 1

100 g (0,25 Mol) Polyisocyanat 1 wird bei 20° C vorgelegt. Man tropft eine Mischung aus 15 g (0,25 Mol) n-Propanol und 2,4 ml Triethylamin innerhalb ca. 10 Minuten zu, wobei die Temperatur von 20°C nicht überschritten wird. Das Reaktionsgemisch wird bei Raumtemperatur nachgerührt bis die CO₂ -Entwicklung beendet ist ( ca. 60 Minuten). Jetzt wird noch 1 Stunde bei 50°C gerührt. Man erhält ein klares Produkt mit einem NCO-Gehalt von 16,5 Gew.-%. Das gemessene CO₂- Volumen lag bei 4,3 Liter, was einer Gasausbeute von 80,4 % entspricht. Das Rohprodukt wird entgast und zur Entfernung des Katalysators Triethylamin bei 20- 25 mbar 2 Stunden bei 50- 60° C gehalten.

IR- und NMR- spektroskopische Untersuchungen belegen, daß das Endprodukt im Wesentlichen aus dem entsprechenden, gesuchten Allophanat- diisocyanat besteht.

### Beispiele 2 bis 9:

Man arbeitet völlig analog Beispiel 1, verwendet jedoch die in der folgenden Tabelle aufgeführten Katalysatoren:

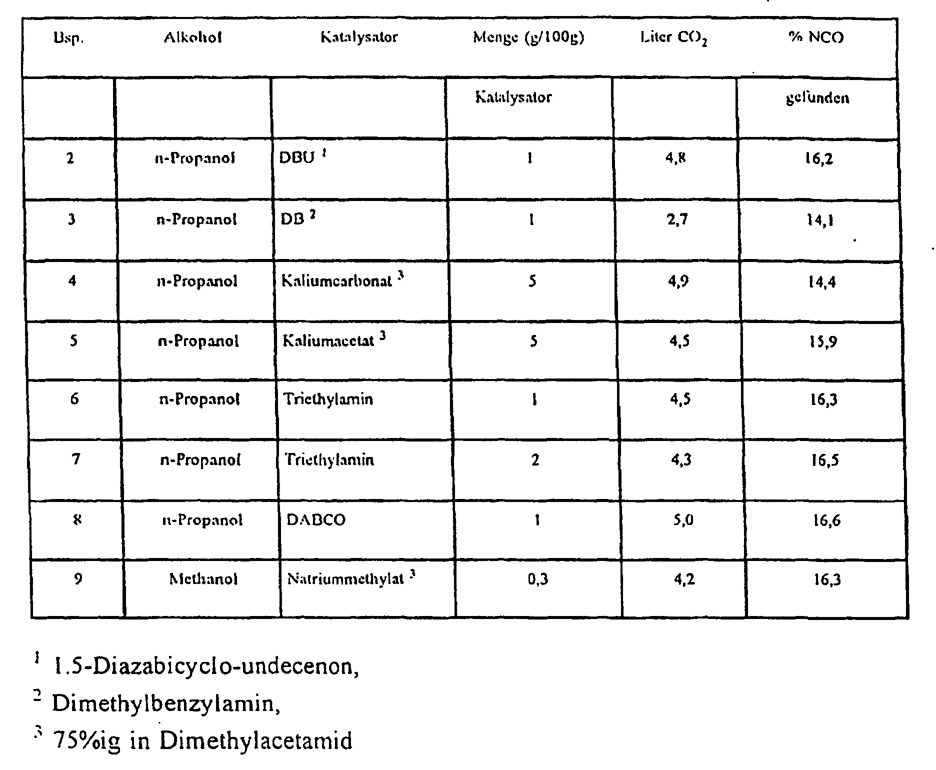

### Beispiele 10-21

Man arbeitet völlig analog Beispiel 1, verwendet jedoch die in der folgenden Tabelle aufgeführten Alkohole (je 0,25 Mole). Als Katalysator wird jeweils 1g Triethylamin pro 100g Reaktionsgemisch verwendet.

| Bsp | Alkohol | Gew.-% NCO | |
|---|---|---|---|
| | | berechnet | gefunden |
| 10 ∘ | Glyzerinketal | 17,2 | 15,9 |
| 11 ∘ | Methoxypolyethylenglykol, MG 350 | 11,9 | 10,5 |
| 12 ∘ | Methoxypolyethylenglykol, MG 500 | 10,4 | 9,2 |
| 13 ∘ | Methoxypolyethylenglykol, MG 750 | 7,6 | 7,2 |
| 14 ∘ | Polyether LB ** | 3,2 | 3,2 |
| 15 | Morpholinoethanol | 17,2 | 15,5 |
| 16 | N,N-Dimethylaminoethanol | 18,9 | 16,2 |
| 17 ∘ | N-Methyldiethanolamin | 17,7 | 16,0 |
| 18 | n-Nonanol | 16,5 | 13,5 |
| 19 | Isopropanol * | 20,1 | 17,8 |
| 20 ∘ | 2.5-Hexandiol * | 17,7 | 13,7 |
| 21 ∘ | Oleinalkohol | 13,5 | 13,1 |

| | | | |
|---|---|---|---|
| * deutlich geringere Gasausbeute | | | |
| ** n-Butanol-gestarteter Polyoxyethylen-polyoxypropylen-polyether vom Molgewicht 2250 | | | |
| ∘ Vergleichsbeispiele | | | |

### Beispiel 22- 25

Man arbeitet völlig analog Beispiel 1, verwendet jedoch je 0,25 Mole der in der folgenden Tabelle aufgeführten Diole bzw. Polyhydroxylverbindungen. Als Katalysator wird jeweils 1g Triethylamin pro 100g Reaktionsgemisch verwendet.

| Bsp. | Alkohol | Gew.-% NCO | |
|---|---|---|---|
| | | berechnet | gefunden |
| 22 ∘ | Diethylenglykol | 18,2 | 16,9 |
| 23 ∘ | 1.4-Butandiol | 18,8 | 17,2 |
| 24 ∘ | Octaethylenglykol | 14,5 | 13,1 |
| 25 ∘ | Hexandiolneopentylglykol-polyadipat, MG 1700 ¹ | 4,1 | 4,2 |

| | | | |
|---|---|---|---|
| ¹ 75%ig in Dimethylacetamid | | | |
| ∘ Vergleichsbeispiele | | | |

### Beispiele 26-28

Die folgenden drei Beispiele wurden analog Beispiel 1 durchgeführt, jedoch unter Verwendung von Ethanol als Alkohol (je 11,5 g) und von je 1,3 g Triethylamin als Katalysator. Variiert wurden die Diisocyanato-oxadiazintrione (je 0,25 Mole).

| Bsp. | Diisocyanato-oxadiazintrion | Gew.-% NCO | |
|---|---|---|---|
| | | berechnet | gefunden |
| 26 | Polyisocyanat 1 | 20,9 | 18,2 |
| 27 | Polyisocyanat 2 | 22,4 | 20,9 |
| 28 | Polyisocyanat 3 * | 17,1 | 16,1 |

| | | | |
|---|---|---|---|
| * 75%ig in Aceton | | | |

## Patentansprüche

1. Verfahren zur Herstellung von (cyclo)aliphatischen und/oder araliphatischen Isocyanatgruppen aufweisenden (Cyclo)Allophanaten der Formel (I) 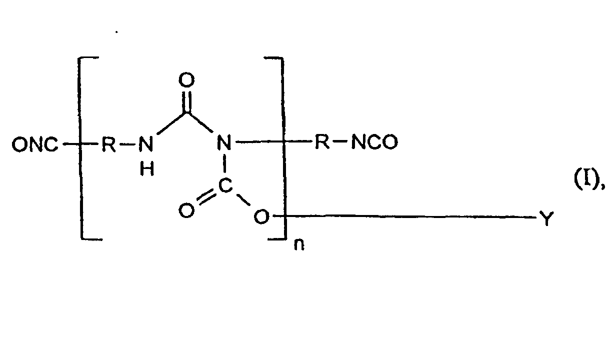 in welcher
R denjenigen Rest bedeutet, wie er durch Abstraktion der Isocyanatgruppen von (cyclo)aliphatischen und araliphatischen Diisocyanaten entsteht,
Y für den ein wertigen Rest eines monofunktionellen Alkohols steht und
n für die Zahl 1, 2, 3, 4 und 5 steht,
**dadurch gekennzeichnet, daß** man Isocyanatgruppen aufweisende (Oligo)-Oxadiazintrione der Formel (II) 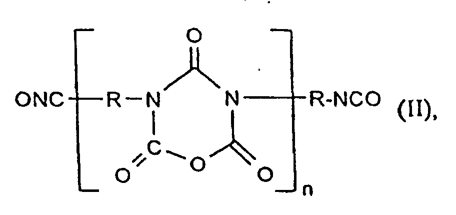 in welcher
R und n die bei Formel (I) angegebene Bedeutung haben in Gegenwart von tertiären Aminen eines pKa-Wertes > 6, Alkali-, Erdalkalisalzen von Kohlensäure, Mono-, Di-, Tricarbonsäuren, Alkoholaten oder Phenolaten als basische Katalysatoren mit primären oder sekundären monofunktionellen Alkoholen umsetzt.

2. Verfahren zur Herstellung von aliphatischen, Isocyanatgruppen aufweisenden Allophanaten gemäß Anspruch 1, **dadurch gekennzeichnet, daß** der Rest R für einen gegebenfalls durch Ethergruppen unterbrochenen und/oder durch Methylgruppen substituierten geradkettigen Rest mit 4-12 Kohlenstoffatomen steht.

3. Verfahren zur Herstellung von aliphatischen, cycloaliphatischen und araliphatischen Isocyanatgruppen aufweisenden Allophanaten gemäß Anspruch 1, **dadurch gekennzeichnet, daß** als Alkohole gegebenenfalls tertiäre. Stickstoff enthaltende primäre Alkohole der Funktionalität 1 und des Molgewichtsbereiches von 32 bis 6000 verwendet werden.

## Claims

1. Process for preparing (cyclo)aliphatic and/or araliphatic isocyanate group-containing (cyclo)allophanates of the formula (I) 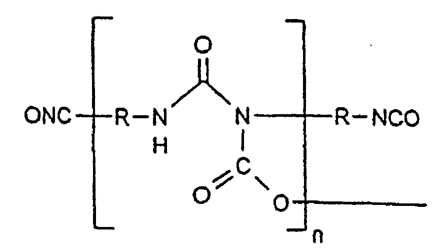 wherein
R denotes the residue obtained by abstracting the isocyanate groups from (cyclo)aliphatic and araliphatic diisocyanates,
Y represents the monovalent residue of a monofunctional alcohol and
n is 1, 2, 3, 4 or 5,
**characterized in that** isocyanate group-containing (oligo)oxadiazinetriones of the formula (II) 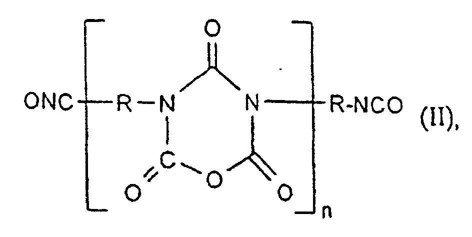 wherein
R and n are as defined for formula (I) are reacted in the presence of tertiary amines having a pKa value of > 6, alkali metal salts, alkaline earth metal salts of carbonic acid, mono-, di-, tricarboxylic acids, alcoholates or phenolates as basic catalysts with primary or secondary monofunctional alcohols.

2. Process for preparing aliphatic, isocyanate group-containing allophanates according to Claim 1, **characterized in that** the residue R represents a radical having 4-12 carbon atoms which is optionally interrupted by ether groups and/or substituted by methyl groups.

3. Process for preparing aliphatic, cycloaliphatic and araliphatic, isocyanate group-containing allophanates according to Claim 1, **characterized in that** primary alcohols optionally containing tertiary nitrogen, having a functionality of 1 and from the molar weight range from 32 to 6000 are used as alcohols.

## Revendications

1. Procédé pour la préparation de (cyclo)allophanates à groupes isocyanate (cyclo)aliphatiques et/ou araliphatiques, répondant à la formule (I) 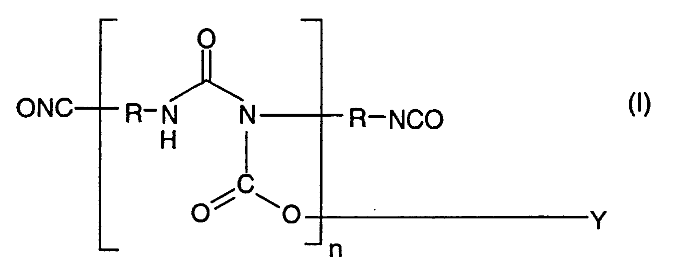 dans laquelle
R représente le radical d'un diisocyanate (cyclo)aliphatique ou araliphatique tel qu'obtenu par élimination des groupes isocyanate,
Y représente le radical monovalent d'un alcool monofonctionnel, et
n est égal à 1, 2, 3, 4 ou 5,
**caractérisé en ce que** l'on fait réagir des (oligo)oxadiazinetriones à groupes isocyanate de formule (II) 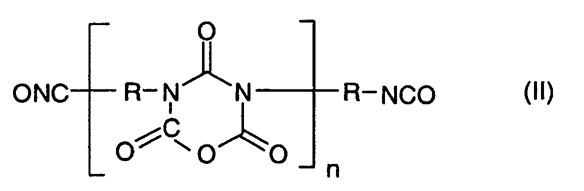 dans laquelle
R et n ont les significations indiquées en référence à la formule (I) avec des alcools monofonctionnels primaires ou secondaires en présence d'amines tertiaires ayant une valeur de pKa supérieure à 6, de sels alcalins, alcalino-terreux de l'acide carbonique, d'acides mono-, di-, tri-carboxyliques, d'alcoolates ou de phénolates, en tant que catalyseurs basiques.

2. Procédé pour la préparation d'allophanates à groupes isocyanate aliphatiques selon la revendication 1, **caractérisé en ce que** R représente un radical à chaîne droite en C₄ à C₁₂ éventuellement interrompu par des groupes éther et/ou substitué par des groupes méthyle.

3. Procédé pour la préparation d'allophanates à groupes isocyanate aliphatiques, cycloaliphatiques ou araliphatiques selon la revendication 1, **caractérisé en ce que** les alcools utilisés sont des alcools primaires monofonctionnels de poids moléculaires 32 à 6000 contenant le cas échéant de l'azote tertiaire.
